(19) Europäisches Patentamt
European Patent Office
Office européen des brevets

(11) EP 0 682 119 B1

(12) EUROPEAN PATENT SPECIFICATION

(45) Date of publication and mention of the grant of the patent:
**03.03.1999 Bulletin 1999/09**

(51) Int Cl.[6]: **C12Q 1/32**, C12Q 1/02, C12N 9/04, C12N 1/20

(21) Application number: **95110124.5**

(22) Date of filing: **18.09.1991**

# (54) Myo-inositol dehydrogenase

Myo-Inositoldehydrogenase

Dehydrogenase myo-inositol

(84) Designated Contracting States:
**AT BE CH DE DK ES FR GB GR IT LI LU NL SE**

(30) Priority: **18.09.1990 JP 249775/90**
**18.09.1990 JP 249776/90**

(43) Date of publication of application:
**15.11.1995 Bulletin 1995/46**

(62) Document number(s) of the earlier application(s) in accordance with Art. 76 EPC:
**91308520.5 / 0 477 001**

(73) Proprietor: **ASAHI KASEI KOGYO KABUSHIKI KAISHA**
**Osaka (JP)**

(72) Inventors:
- **Ueda, Shigeru**
  **Tagata-gun, Shizuoka (JP)**
- **Takahashi, Mamoru**
  **Sunto-gun, Shizuoka (JP)**
- **Misaki, Hideo**
  **Tagata-gun, Shizuoka (JP)**
- **Imamura, Shigeyuki**
  **Tagata-gun, Shizuoka (JP)**
- **Matsuura, Kazuo**
  **Tagata-gun, Shizuoka (JP)**

(74) Representative: **Woods, Geoffrey Corlett**
**J.A. KEMP & CO.**
**14 South Square**
**Gray's Inn**
**London WC1R 5LX (GB)**

(56) References cited:
- **JOURNAL OF BIOLOGICAL CHEMISTRY, vol. 254, no. 16, 1 August 1979 WASHINGTON DC USA, pages 7684-7690, R. RAMALEY ET AL. 'Purification and properties of Bacillus subtilis inositol dehydrogenase.'**

Remarks:
The file contains technical information submitted after the application was filed and not included in this specification

EP 0 682 119 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This invention relates to a myo-inositol dehydrogenase, a process for its production, a Bacillus capable of producing it and a culture of the Bacillus.

Myo-inositol is the one of nine isomers of inositol and is a stable cyclic alcohol. In humans myo-inositol is supplied in an amount of approximately one gram per day from daily meals and approximately 2 grams from renal biosynthesis.

The blood level of myo-inositol is controlled to a constant level by a balance of intake into cells and renal excretion, reabsorption and oxidation.

The plasma level of myo-inositol is increased by a renal functional disorder [Clinical Chem., 24(11); 1448-1455 (1988)]. The renal function can be monitored by measuring the level of myo-inositol in the blood.

Heretobefore the amount of myo-inositol in a specimen has been measured by gas-chromatography and high performance liquid chromatography (HPLC). These measuring methods have a number of disadvantages, for example the specimen has to be previously treated and the operation needs a complex treatment. Hence large numbers of assays for clinical chemistry was impossible. The normal standard level of myo-inositol in blood plasma is quite low, such as approximately 30 μmol/l [Proceedings of Japan Clinical Chemistry Annual Meeting No. 28 (1988)]. A simple and highly sensitive assay method is desired.

An enzymatic cycling method for assaying trace amount of substrates or enzyme activity using two types of enzyme to amplify the sensitivity is known. Among them, NAD-cycling, CoA-cycling and ATP-cycling are known but are almost never applied to routine assay methods for clinical chemistry due to complex operations.

Myo-inositol dehydrogenases are produced by the following microorganisms, (described in Enzyme Handbook (Asakura Publishing Co., Tokyo)).

Aerobacter aerogenes [J. Biol. Chem., 241, 800-806 (1966)]; Klebsiella pneumoniae, Serratia marcescens, Cryptococcus melibiosum [Biochim, Biophys, Acta., 293, 295-303 (1973)]; and Bovine brain [Biochem. Biophys. Res. Comm., 68, 1133-1138 (1976)]; Bacillus sp. No. 3 (product of Toyo Jozo Co.).

Among these, Aerobacter aerogenes, Klebsiella pneumoniae and Serratia marcescens are known as etiologic microorganisms for pneumoniae and opportunistic infections (Standard Microbiology., p. 209-212, Igaku Shoin Publ., Tokyo) with inveterate infection resistant to chemotherapeutics and antibiotics. Culturing these microorganisms on an industrial scale is substantially impossible. The Km-value of myo-inositol dehydrogenase produced by yeast Cryptococcus melibiosum is approx. 11.0 mM for myo-inositol and approx. 0.07 mM for NAD. These values of Km are too high to get a sufficient reaction rate in an enzymatic cycling assay. (Enzyme Handbook, p.6).

Ramaley *et al* (J. Biol. Chem., 254, 7684-7690) discloses the purification and properties of *Bacillus subtilis* inositol dehydrogenase. The enzyme has an apparent molecular weight of 155,000 to 160,000 as determined by sucrose density gradient centrifugation, and it is comprised of four subunits, each having a molecular weight of 39,000 as determined by sodium dodecyl sulfate-polyacrylamide gel electrophoresis. The isoelectric point of the enzyme is 4.4 as determined by column isoelectric focussing. The enzyme shows the highest $v_{max}$ and lowest $K_m$ with myo-inositol as substrate but does not react with scyllo-inositol; it also reacts with the α anomer (but not the β anomer) of D-glucose and with D-xylose.

We have found a new myo-inositol dehydrogenase (EC. 1.1.1.18) which can be used in a cycling reaction with thio-NADP group coenzymes.

The present invention provides a myo-inositol dehydrogenase which has the following properties:

(1) substrate specificity for myo-inositol and catalyses the reaction:

$$\text{myo-inositol} + \text{NAD} \rightleftharpoons \text{myo-inosose} + \text{reduced NADH}$$

(2) molecular weight : 130,000 ± 15,000 (gelfiltration method by TSK gel G 3000 SW)
(3) isoelectric point: pH 4.5 ± 0.5
(4) Km-value:

| | |
|---|---|
| Km value for myo-inositol | 0.64 mM |
| Km value for NAD | 0.004 mM |

(5) optimum pH: approx. 9.5
(6) pH-stability: more than 80% of remaining activity at a pH of from 6.5 to 9.0
(7) heat stability: more than 95% of remaining activity at temperatures up to 60°C.

The present invention also provides a process for the production of a myo-inositol dehydrogenase of the invention which comprises culturing a myo-inositol dehydrogenase producing microorganism which is Bacillus sp. No. 3 (FERM BP-3013) or a mutant thereof, and isolating said myo-inositol dehydrogenase from the cultured mass.

The present invention further provides Bacillus sp. No. 3 (FERM BP-3013) or a mutant thereof which is capable of producing a myo-inositol dehydrogenase according to the invention.

The present invention additionally provides a culture of Bacillus sp. No. 3 (FERM BP-3013), or a mutant thereof which is capable of producing a myo-inositol dehydrogenase according to the invention, in a culture medium which comprises a source of assimilable carbon, a source of assimilable nitrogen and mineral salts and which is free of other microorganisms.

The myo-inositol dehydrogenase of the invention may be used in a method of assaying a specimen for myo-inositol which comprises reacting the specimen with:

a) the myo-inositol dehydrogenase using one of the thionicotoinamide adenine dinucleotide phosphate group (thio-NADP group) or thionicotoinamide adenine dinucleotide group (thio-NAD group) and one of the nicotinamide adenine dinucleotide phosphate group (NADP group) or nicotinamide adenine dinucleotide group (NAD group) as coenzymes,
b) $A_1$, and
c) $B_1$;

to effect the cycling reaction:

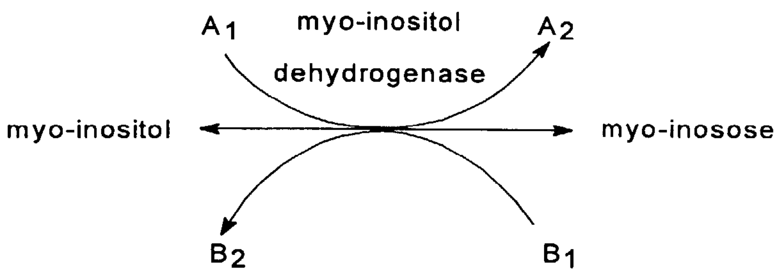


wherein

$A_1$ is a thio-NADP, thio-NAD, NADP or NAD group compound,
$A_2$ is a reduced form of $A_1$,
$B_1$ is a reduced NADP or reduced NAD group compound when $A_1$ is a thio-NADP or thio-NAD group compound or $B_1$ is a reduced thio-NADP or reduced thio-NAD group compound when $A_1$ is a NADP or NAD group compound, and
$B_2$ is an oxidized form of $B_1$; and
measuring the amount of $A_2$ or $B_1$ generated or consumed in the cycling reaction.

This assay method depends on the difference in the maximum absorption of a reduced thio-NAD(P) group, which is an analogue of NAD(P), at approximately 400 nm, and that of a reduced NAD(P) group at approximately 340nm. In the enzymatic cycling reaction using myo-inositol dehydrogenase, a thio-NAD(P) group is used as one of the two coenzymes, and the change in amount of any of the coenzymes is measured, for example by the difference in maximum absorptia of both reduced coenzymes. Thus the amount of myo-inositol in a specimen can be most precisely measured.

Brief Description of the Drawings

Figure 1: heat stability of the novel myo-inositol dehydrogenase
Figure 2: optimum temperature of the myo-inositol dehydrogenase
Figure 3: pH-stability of the myo-inositol dehydrogenase
Figure 4: optimum pH of the myo-inositol dehydrogenase

Figures 5-8: assay curve of myo-inositol.

Detailed Description of the Invention

We have screened for non-infectious microorganisms which can produce a myo-inositol dehydrogenase which has higher activity, low Km-value on substrate myo-inositol and NAD, is stable, easily purified. We have isolated a microorganism designated Bacillus sp. No. 3 from a soil sample in a hot spring area, Atagawa, Higashi-Izu-cho, Kamo-gun, Shizuoka-ken, Japan.

Myo-inositol dehydrogenase produced by Bacillus sp. No. 3 has low Km-values for myo-inositol and NAD at pH 8.5 of approx. 0.6 mM and 0.04 mM, respectively, with high reactivity. Moreover remaining activity after treating at 60°C for 15 minutes in buffer solution is over approx. 95%. The myo-inositol dehydrogenase is a novel enzyme which can act with a coenzyme NAD(P) group or a thio-NAD(P) group.

The novel myo-inositol dehydrogenase can also be produced by isolating the enzyme from other myo-inositol dehydrogenase producing microorganisms belonging to the genus Bacillus.

The present myo-inositol dehydrogenase producing microorganism, Bacillus sp. No. 3, belongs to the genus Bacillus, and is a preferred microorganism.

The taxonomical properties of this strain are as follows:

(a) Morphological properties:

Round edge with straight or slightly curved bacillus. Sizes are 0.5 ~ 07 x 1.5 - 3.5μm. Peritrical movement. Spore formations in an edge or subedge with sizes in 0.8 x 1.0 ~ 2.0 μm of elliptical to oval spores are observed. Microbial cells are swollen with spores. No polymorphism.

(b) Growth on various media:

Observations on various media, cultured for 1-2 days at 50-52°C, are as follows:

1. Nutrient agar plate medium: round and convex colonies. Smooth wettish surface with round edge. Ocherous or plate ocherous. No formation of soluble pigment.
2. Nutrient agar slant medium: good growth with filiform. Ocherous to plate ocherous. No formation of soluble pigment.
3. Liquid medium (aqueous peptone) : good growth with uniform turbidity.
4. Litmus milk medium : weakly acidic after 4-5 days. GC mole % in DNA : 41.9 mole % (HPLC method)
   Main isoprenoid quinone : MK - 7

| (c) Physiological properties (+= positive, (+)=weakly positive, -= negative, NT= not tested) | | |
|---|---|---|
| Gram-strain | | + |
| KOH reaction | | - |
| Capsule formation | | - |
| Acid fastness stain | | - |
| OF-test (High Leifson) | | NT |
| OF-test (nitrogen source: $NH_4H_2PO_4$) | | F |
| Aeroboc growth | | + |
| Anaerobic growth | | + |
| Growth temperature | 70 °C | - |
| | 60 °C | + |
| | 37 °C | + |
| | 30 °C | - |
| Halotolerant NaCl conc. | % | |
| | 0% | + |
| | 3% | + |
| | 5% | - |
| Growth pH | | |
| | pH 5.6 | - |

(continued)

| (c) Physiological properties (+= positive, (+)=weakly positive, -= negative, NT= not tested) | | |
|---|---|---|
| | pH 6.2 | + |
| | pH 9.0 | + |
| Gelatin hydrolysis | | - |
| Starch hydrolysis | | (+) |
| Casein hydrolysis | | - |
| Esculin hydrolysis | | + |
| Cellulose hydrolysis | | - |
| Tyrosine hydrolysis | | - |
| Arginine hydrolysis | | - |
| Catalase production | | + |
| Oxidase production | | + |
| Lecithinase production | | - |
| Urease production (SSR) | | - |
| Urease production (Chris) | | - |
| Indol production | | - |
| $H_2S$ production (detection: lead acetate paper) | | - |
| Acetoin production ($K_2HPO_4$) | | - |
| Acetoin production (NaCl) | | - |
| MR test | | - |
| Nitrate reduction | | |
| Gas detection | | - |
| $NO_2^-$ | | - |
| $NO_3^-$ | | + |
| Utilization of Simmons medium | | |
| Citrate | | - |
| Malate | | - |
| Maleate | | - |
| Malonate | | - |
| Propionate | | - |
| Gluconate | | - |
| Succinate | | - |
| Utilization of Christenssen medium | | |
| Citrate | | + |
| Malate | | - |
| Maleate | | - |
| Malonate | | - |
| Propionate | | + |
| Gluconate | | - |

(continued)

| (c) Physiological properties (+= positive, (+)=weakly positive, -= negative, NT= not tested) | |
|---|---|
| Succinate | - |
| Gas production from glucose | - |
| Acid formation from sugar | |
| Adonitol | - |
| L (+) arabinose | - |
| Cellobiose | + |
| Dulsitol | - |
| Meso-erythritol | - |
| Fructose | + |
| Fucose | + |
| Galactose | + |
| Glucose | + |
| Glycerin | + |
| Inositol | + |
| Inulin | + |
| Lactose | + |
| Maltose | + |
| Mannitol | + |
| Mannose | + |
| Melezitose | - |
| Melibiose | + |
| Raffinose | - |
| Rhamnose | + |
| D-ribose | + |
| Salicin | + |
| L-sorbose | - |
| Sorbitol | - |
| Starch | + |
| Saccharose | + |
| Xylose | - |
| Trehalose | + |

According to the above taxonomical properties, the microorganism displays the specific characteristics of Gram positive bacillus, namely, it is 0.5 ~ 0.7 × 1.5 ~ 3.5µm in size, is peritrichal in movement, has spore formation with no polymorphism, promotes fermentative decomposition of glucose and acid formation, is catalase positive and oxidase positive, and is thermophilic and is a facultative anaerobe.

Among Gram-positive bacillus with specific properties of spore formation and aerobic growth, the strain belongs to be genus Bacillus.

According to Bergey's Manual of Systematic Bacteriology, Vol. 2, there are illustrated the following 9 species of Bacillus with growth in high temperature (50 °C).

Bacillus acidocaldarius, B. subtilis, B. badius, B. brevis, B. coagulans, B. licheniformis, B. pantothenticus, B. schegelli and B. stearothermophilus.

Among these, microorganisms growing at anaerobic condition are B. coagulance and B. licheniformis.

Taxonomic properties of Bacillus in comparison with those of the present strain, according to Bergey's Manual, are illustrated by comparing Bacillus coagulans (hereinafter designated C) and Bacillus licheniformis (hereinafter designated L), as follows:

| | |
|---|---|
| + = | positive |
| (+) = | weakly positive |
| - = | negative |
| d = | not identified as + or - |
| ND = | no data |

| | | C | L | The present strain |
|---|---|---|---|---|
| Oxidase production | | - | d | + |
| Swelling with spore | | d | - | + |
| Anaerobic growth | | + | + | + |
| Acetoin production | | + | + | - |
| Glucose (acid) | | + | + | + |
| L-arabinose (acid) | | + | + | + |
| Xyrose | | d | + | - |
| Mannitol | | d | + | + |
| Casein hydrolysis | | d | + | - |
| Gelatin hydrolysis | | d | + | - |
| Starch hydrolysis | | - | + | (+) |
| Citrate utilization | | + | + | - |
| Propionate utilization | | d | + | - |
| Tyrosine hydrolysis | | - | + | - |
| LV-reaction | | - | + | - |
| Indol production | | - | + | - |
| Halotolerant | 2 % | + | + | + |
| | 5 % | - | + | - |
| | 7 % | - | + | - |
| | 10 % | - | ND | - |
| Growth tremperature | | | | |
| | 40 °C | + | + | + |
| | 50 °C | + | + | + |
| | 55 °C | + | + | + |
| | 60 °C | ND | ND | + |
| | 70 °C | - | - | - |
| Nitrate reduction | | d | + | - |
| GC mole % in DNA | | 44.5 (Type) 44.3 ~50.3 | 46.4 (Type) 42.9 ~49.9 | 41.9 |

According to the above comparison, the present strain No. 3 has many identical properties with Bacillus coagulans but has specific differences as to acetoin production and GC mole % in DNA. Further an observation on Litmus milk medium is different. (not mentioned in the above comparison).

Accordingly, the present strain has been designated Bacillus sp. No. 3 and has been deposited at the Fermentation Research Institute and assigned deposite No. FERM BP-3013.

According to the present invention the production of the enzyme, myo-inositol dehydrogenase is obtained by culturing a microorganism belonging to genus Bacillus in suitable medium.

A Preferred example of the above myo-inositol dehydrogenase producing microorganism is Bacillus sp. No. 3. Since taxonomical properties of bacteria are in general easily mutated, natural variants or artificial mutants produced by conventional artificial mutation treatment for example, ultraviolet irradiation, radiation or mutagen such as N-methyl-N-nitro-N-nitrosoguanidine and ethyl methansulfonate, which belong to the genus Bacillus and which produce a myo-inositol dehydrogenase according to the invention can be used in the present invention.

The above microorganism can be cultured in conventional culture conditions for bacteria. In the case of producing enzyme from Bacillus sp. No.3 since myo-inositol dehydrogenase is inducible by myo-inositol, the culture is preferably done in a medium containing myo-inositol 0.5 ~ 5% which causes 10 ~ 300 times more myo-inositol dehydrogenase to be produced.

As the examples of medium other than myo-inositol, a nutrient medium containing an assimilable carbon source, a digestible nitrogen source and if required inorganic salts can be used.

Examples of carbon sources are glucose, fructose or saccharose which can be used in combination or alone. Examples of digestible nitrogen sources are peptone, meat extract or yeast extract which can be used in combination or alone. Further according to the requirement, phosphate, magnesium salt, calcium salt, potassium salt, sodium salt or various heavy metal salts such as ferric salt or manganese salt can be added. Other known assimilable carbon sources and digestible nitrogen salts can also be used.

Culturing can be by shake culture or aeration culture with agitation, and for industrial production, submerged aeration culture with agitation is preferable. The Culture temperature can be altered to within the range in which the enzyme is produced and is generally 40 ~ 60 °C, preferably approx. 50°C.

The Culture time depends on the condition of culture, and can be set up for maximum production of the enzyme, and is generally 1 ~ 2 days.

The composition of medium, medium condition, culture temperature, agitation speed and air flow should of course be adjusted to give preferably conditions for enzyme production. Anti-foaming agents such as silicon oil and vegetable oil can also be used if required.

Since the thus produced myo-inositol dehydrogenase exists as an enzyme with the bacterial cells, cultured cells are collected by means of filtration or centrifugation, and the bacterial cells are broken up by means of mechanical destruction such as ultra sonication, French press treatment, glass beads treatment or freezing-thawing, or enzymatic digestion such as lysozyme treatment, to obtain a crude extract.

Further purified myo-inositol dehydrogenase can be obtained by known conventional purification methods of isolation of protein or enzyme. For example, the enzyme can be precipitated by a salting-out method by adding ammonium sulfate or sodium sulfate to the crude extract. The precipitated enzyme can be further purified by a combination of chromatographic techniques using molecular sieves and resins, electrophoresis or ultra-centrifugation.

The purification can be aided by considering the nature of myo-inositol dehydrogenase. For example, the above precipitate dissolved in water or buffer solution can be dialysed, if required, using a semi-permeable membrane, and subjected to chromatography using ionexchange resins such as DEAE-cellulose*, DEAE-Sephacel*, DEAE-Sepharose*, DEAE-Sephadex*, Q-Sepharose* (Pharmacia Corp., trade name), DEAE-Toyoperal* (Toso Corp.) or hydroxy apatite, hydrophobic chromatographic resin such as octyl -Sepharose or phenyl-Sepharose (Pharmacia Corp.) and other affinity chromatographic resins. Further molecular sieve chromatography using gel-filtration agent such as Sephadex G-100* or Sephacryl S-200* can be done. If required desalting by means of semi-permeable membrane can also be done. Purified myo-inositol dehydrogenase powder preparation can be prepared by lyophilization by the addition of 0.05 ~ 10 % of stabilizing agent for example sugars such as mannitol, saccharose or sorbitol, amino acids such as glutamate or glycine, and peptide or such as bovine albumin.

Typically a Myo-inositol dehydrogenase of the present invention has the following properties.

1. Substrate specificity:

| | |
|---|---|
| myo-inositol | 100 % |

* : Believed to be a Registered Trade Mark in one or more designated states.

(continued)

| | |
|---|---|
| glucose | 0 |
| fructose | 0 |
| galactose | 0 |
| sorbitol | 0 |
| mannose | 0 |
| maltose | 0 |
| saccharose | 0 |
| lactose | 0 |

2. Enzyme action:

The enzyme catalyzes essentially a reaction of myo-inositol and NAD to generate myo-inosose and reduced NADH, as shown below.

myo-inositol + NAD $\rightleftarrows$ myo-inosose * + reduced NADH

*(2, 4, 6/3, 5-pentahydroxy cyclohexanone)

3. Molecular weight:

130,000 ± 15,000

Measured by TSK-gel G 3000 SW 8 (Toso Co., 0.75 × 60 cm).

Elution : 0.1 M phosphate buffer (pH 7.0) containing 0.2 M NaCl.

Standard : following molecular markers (Oriental yeast Co) are used.

| | |
|---|---|
| M. W. 12,400 | Cytochrome C |
| M. W. 32,000 | adenylate kinase |
| M. W. 67,000 | bovine albumin |
| M. W. 142,000 | lactate dehydrogenase |
| M. W. 290,000 | glutamate dehydrogenase |

4. Isoelectric point:

pH 4.5 ± 0.5

Measured by elelctrofocussing using carrier ampholite at 4°C, 700 V, for 40 hours. The activity of a fraction of each enzyme is measured.

5. Km-value: 0.64 mM (myo-inositol), 0.004 mM (NAD)

Km-value for myo-inositol is measured in various concentrations of myo-inositol in a reaction mixture of:

100 mM Tris-HCl buffer (pH 8.5)
5 U diaphorase (Toyo Jozo Co.)
1 mM NAD (Oriental yeast Co.)
0.025 % NBT (Wako Pure Chem. Co.)

In the reaction mixture, NAD is replaced by 15 mM myo-inositol, and the concentration of NAD is varied to measure the Km-value for NAD.

The results are as shown above.

Further in the reaction mixture, 1 mM NAD is replaced by 1 mM thio-NAD (Sigma Co.) and the Km-value for myo-inositol is measured. The result is as shown below.

Km-value: 10.0 mM (myo-inositol)

In the reaction mixture, 1 mM thio-NAD is replaced by 150 mM myo-inositol, and the Km-value for thio-NAD

is measured.

Km-value : 0.17 mM (thio-NAD)
Km-value in the reaction of NADP and myo-inositol are measured. The results are as shown below.
Km-value : 0.19 mM (NADP)
Km-value : 30.91 mM (myo-inositol)
Km-values in the reaction of the thio-NADP and myo-inositol are as shown below.
Km-value : 2.54 mM (thio-NADP)
Km-value : 179.62 mM (myo-inositol)

As clearly shown hereinabove, the present enzyme can be reacted using NAD(P) and thio-NAD(P) as the coenzymes.

6. Optimum pH:

In an assay method for enzyme activity as illustrated hereinafter, 100 mM Tris-HCl buffer (pH 8.5) in the reaction mixture is replaced by 100 mM phosphate buffer (pH 6.5-8.0, -○-), 100 mM Tris-HCl buffer (pH 8.9-9.0, -□ -) and 100 mM glycine-NaOH buffer (pH 9.0-10.0,- ■-), and incubated. The results are shown in Fig. 4.
Maximum activity is observed at approx. pH 9.5.

7. pH-stability:

The residual activity of the enzyme (1 U/ml, 40 mM buffer solution) is measured in various buffer solutions, i. e. acetate buffer (pH 4.5-6.0, - ▲-) ; phosphate buffer (pH 6.0-8.0. - ○-) ; Tris-HCl buffer (pH 8.0-9.5, - □-) and glycine-NaOH buffer (pH 9.0-10, -■-) after heating at 50°C for 15 mins. The enzyme is stable at pH 6.5-9.0 with a residual activity of over 80 %, as shown in Fig.3.

8. heat-stability:

The enzyme, dissolved in 20 mM Tris-HCl buffer (pH 7.0), to produce a 1 U/ml solution, is incubated for 15 min. at various temperatures, and the residual activity is measured.
The results are as shown in Fig. 1. The enzyme is stable up to 60°C, preferably up to 65°C, while maintaining a residual activity of over 95 %.

9. Optimum temperature:

The enzyme activity is measured at 35, 40, 50, 55, 60 and 65°C, respectively, in 100 mM Tris-HCl buffer (pH 8.5) according to the assay method illustrated hereinafter. The reaction was stopped in each case after 10 mins. incubation by adding 0.1 N HCl (2 ml), whereupon the optical absorption was measured at 550nm. The enzyme shows maximum activity at 60 °C as shown in Fig. 2.

10. Assay method of myo-inositol dehydrogenase

(1) Reaction mixture:

100 mM Tris-HCl buffer (pH 8.5)
15 mM Myo-inositol (Wako Pure Chem. Co)
1 mM NAD (Oriental Yeast Co.)
5 U Diaphorase (Toyo Jozo Co.)
0.025 % NBT (Wako Pure Chem. Co.)

(2) Enzyme Assay:

The above reaction mixture (1 ml) is incubated in a small test tube at 37°C for 5 mins. Diluted enzyme solution (0.02 ml) is added and stirred to initiate the reaction. After exactly 10 mins., 0.1 N HCl (2.0 ml) was added and stirred to stop the reaction. Absorption at 550 nm ($A_{550}$ nm) is measured to obtain absorption $A_1$. The assay was repeated

using the above reaction mixture except that myo-inositol was not included. The mixture is also treated in the same manner as described above and its absorption Ao was measured.

(3) Calculation of enzyme activity:

$$U/ml = \frac{(A_1 - A_0)}{18.3} \times \frac{1}{10} \times \frac{3.02}{0.02} \times Z$$

wherein

18.3 : molecular absorption coefficient ($cm^2/\mu mol$)
Z : dilution factor

In the enzymatic reaction hereinbefore illustrated, $A_1$ or $B_2$ is of the thio-NADP-group, thio-NAD group, NADP group or NAD group of coenzyme. Examples of the thio-NADP group or thio-NAD group are thionicotinamide adenine dinucleotide phosphate (thio-NAD) and thionicotinamide hypoxanthine dinucleotide phosphate or thionicotinamide adenine dinucleotide (thio-NAD) and thionicotinamide hypoxanthine dinucleotide. Examples of the NADP group or NAD group are nicotinamide adenine dinucleotide phosphate (NADP), acetylpyridine adenine dinucleotide phosphate (acetyl NADP) or nicotinamide hypoxanthine dinucleotide phosphate (deamino NADP) and nicotinamide adenine dinucleotide (NAD), acetylpyridine adenine dinucleotide (acetyl NAD) and nicotinamide hypoxanthine dinucleotide (deamino NAD).
When $A_1$ is of the thio-NAD(P) group, $B_1$ is of the NAD(P)H group, and when $A_1$ is of the NAD(P) group, B, is of the thio-NAD(P)H group. Hence at least one will be a thio-type coenzyme.
Further when thio-NAD group and NAD group compounds are used as coenzymes of myo-inositol dehydrogenase, any of thio-NAD group and NAD group hereinabove illustrated can be selected. Furthermore, when thio-NAD (P) group and NAD(P) group compounds are used as coenzymes of the enzyme, any of thio-NAD group or thio-NADP group and NAD group or NADP group can be selected.
In a composition for an assay of myo-inositol the concentration of $A_1$ and $B_1$ is 0.02-100 mM, preferably 0.05-20 mM, and the concentration of myo-inositol dehydrogenase is 5-1000 U/ml, preferably 2-500 U/ml. The amount exceeding the above range is acceptable.
$A_1$ and $B_1$ are used in excess as compared with myo-inositol and are in excess as compared with the Km-value (mM) of myo-inositol dehydrogenase for $A_1$ and $B_1$. Specifically a 20-10,000 times molar excess relative to myo-inositol is preferred.
A second dehydrogenase which does not act on myo-inositol and provides a reaction from $B_2$ to $B_1$ may be used as a fourth component (4) in a cycling assay. The second dehydrogenase and a substrate for the said second dehydrogenase are combined to effect a cycling reaction of the formula (II). In this formula a reaction $B_1 \rightarrow B_2$, and an amount of $A_2$ generated is measured.

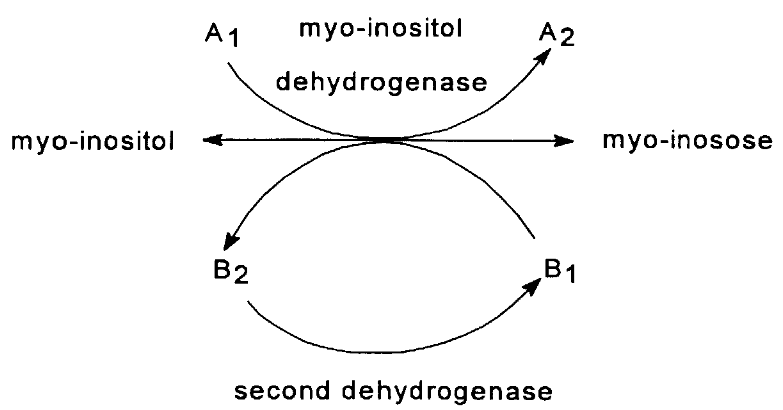


(II)

wherein $A_1$ is thio-NADP group, thio-NAD group NADP group or NAD group, $A_2$ is a reduced form of $A_1$, when $A_1$ is thio-NADP group or thio-NAD group, $B_1$ is reduced NADP group or reduced NAD group and when $A_1$ is NADP

groupor NAD group, $B_1$ is reduced thio-NADP group or reduced thio-NAD group, and wherein $B_2$ is an oxidized form of $B_1$, and the reaction from $B_2$ to $B_1$ is an enzymatic reaction which regenerate $B_1$ by an action of the second dehydrogenase with coenzyme of $B_2$. The second dehydrogenase is added as a supplement for regenerating the $B_1$. Thus the amount of $B_1$ can be reduced or replaced by $B_2$ or a mixture of $B_1$ and $B_2$. In this case the amount of $B_1$ or/and $B_2$ is not limited, but is generally below 1/10 mole as of $A_1$, preferably 1/50~ 1/1000 mole, or less.

In a composition for an assay of myo-inositol using the component (4) the concentration of $A_1$ is 0.02 ~ 100 mM, preferably 0.05 ~ 20 mM, the concentration of $B_2$ or/and $B_1$ is 0.05 ~ 5000 μM, preferably 5 ~ 500 μM, and the concentration of myo-inositol dehydrogenase is 5 ~ 1000 U/ml, preferably 20 ~ 500 U/ml. The concentration of the second dehydrogenase is set up 20 times (U/ml) or more as compared with the Km-value of the second myo-inositol dehydrogenase for $B_2$, for example preferably 1~ 100 U/ml. The substrate for the second dehydrogenase is used in excess, preferably 0.05 ~ 20 mM or more.

Examples of the second dehydrogenase and a substrate for the second dehydrogenase are as follows.

$B_2$ : NAD group or thio-NAD group;
Alcohol dehydrogenase (EC.1.1.1.1) and ethanol,
Glycerol dehydrogenase (EC.1.1.1.6)(E. coli) and glycerol,
Glycerol-3-phosphate dehydrogenase (EC.1.1.1.8) (rabbit muscle) and L-glycerol-3-phosphate,
Maleic dehydrogenase (EC.1.1.1.37) (porcine heart muscle, bovine heart muscle) and L-malate, and
Glyceraldehyde phosphate dehydrogenase (EC.1.2.1.12) (rabbit muscle, liver, yeast, E. coli) and D-glyceraldehyde phosphate and phosphate.

$B_2$ : NADP group or thio-NADP group;
Glucose-6-phosphate dehydrogenase (EC.1.1.1.49) (yeast) and glucose-6-phosphate,
Isocitrate dehydrogenase (EC.1.1.1.42) (yeast, porcine heart muscle) and isocitrate,
Glyoxylate dehydrogenase (EC.1.2.1.17) (Pseudomonas oxalaticus) and CoA and glyoxylate,
Phosphogluconate dehydrogenase (EC.1.1.1.44) (rat liver, beer yeast, E. coli) and 6-phosphogluconate,
Glyceraldehyde dehydrogenase (EC.1.2.1.13) (chlorophyll) and D-glyceraldehyde-3-phosphate and phosphate, and

Benzaldehyde dehydrogenase (EC.1.2.1.7) (Pseudomonas fluorescens) and benzaldehyde.

A third dehydrogenase which does not act on myo-inositol and which provides a reaction from $A_2$ to $A_1$ may be used as a fifth component (5) in a cycling assay. The third dehydrogenase and a substrate for the said third dehydrogenase are combined to effect a cycling reaction of the formula (III). In this formula a reaction for regenerating the $A_1$ is added to the reaction $A_1$ $A_2$, and an amount of $B_1$ decreased is measured.

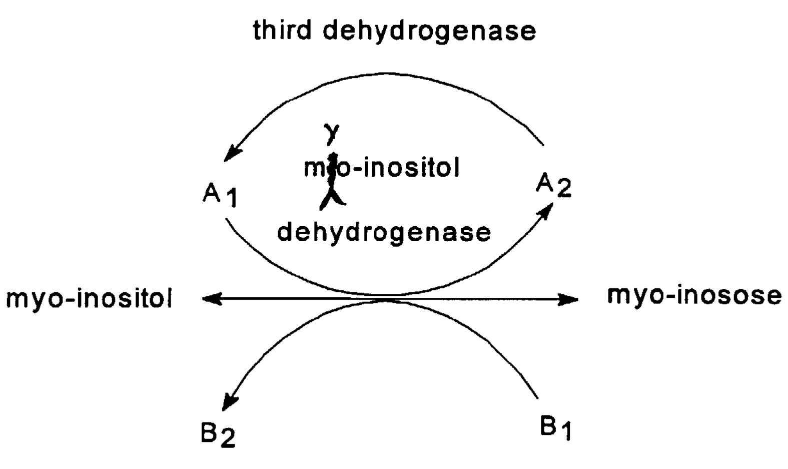


( III )

wherein $A_1$ is thio-NADP group, thio-NAD group, NADP group or NAD group, $A_2$ is a reduced form of $A_1$, when $A_1$ is thio-NADP group or thio-NAD group, $B_1$ is reduced NADP group or reduced NAD group and when $A_1$ is NADP group or NAD group, $B_1$ is reduced thio-NADP group or reduced thio-NAD group, and wherein B2 is an oxidized form of $B_1$, and the reaction from $A_2$ to $A_1$ is an enzymatic reaction which regenerates $A_1$ by an action of the third dehydrogenase with coenzyme of $A_2$. The third dehydrogenase is added as a supplement for regenerating the $A_1$. The amount of $A_1$ can be reduced or $A_1$ can be replaced by $A_2$ or a mixture of $A_1$ and $A_2$. In this case the amount of $A_1$ or/and $A_2$

is not limited, but is generally below 1/10 mole as of $B_1$, preferably 1/50~1/1000 mole, or less.

In a composition for an assay of myo-inositol using the component (5) the concentration of $B_1$ is 0.02 ~ 100 mM, preferably 0.05 ~ 20 mM, the concentration of $A_2$ or/and $A_1$ is 0.05 ~ 5000 μM, preferably 5 ~ 500 μM, and the concentration of myo-inositol dehydrogenase is 5 ~ 1000 U/ml, preferably 20 ~ 500 U/ml. The concentration of the third dehydrogenase is set up 20 times (U/ml) or more as compared with the Km-value of the third myo-inositol dehydrogenase for $A_2$, for example preferably 1~ 100 U/ml. Substrate for the third dehydrogenase is used in excess, preferably 0.05 ~ 20 mM or more.

Examples of the third dehydrogenase and a substrate for the third dehydrogenase are as follows.

$A_1$ : NAD group or thio-NAD group;
Alcohol dehydrogenase (EC.1.1.1.1) and acetoaldehyde,
Glycerol dehydrogenase (EC.1.1.1.6)(E. coli) and dihydroxyacetone
Glycerol-3-phosphate dehydrogenase (EC.1.1.1.8) (rabbit muscle) and dihydroxyacetone phosphate,
Maleic dehydrogenase (EC.1.1.1.37) (porcine heart muscle, bovine heart muscle) and oxaloacetate and
Glyceraldehyde phosphate dehydrogenase (EC.1.2.1.12) (rabbit muscle, liver, yeast, E. coli) and 1, 3-diphospho-D-glycerate.

$A_1$: NADP group or thio-NADP group;
Glucose-6-phosphate dehydrogenase (EC.1.1.1.49) (yeast) and gluconolactone-6-phosphate, and
Glyceraldehyde phosphate dehydrogenase (EC.1.2.1.13) (chlorophyll) and 1,3-diphospho-D-glycerate.

In the reaction medium composition for the cycling assay, two coenzymes are selected by considering their relative activity with myo-inositol dehydrogenase. Thereafter, the pH condition considering the optimum pH of the forward reaction and the reverse reaction is adjusted such that the ratio of the forward reaction rate to reverse reaction rate approaches 1:1

Myo-inositol dehydrogenase produced by Bacillus sp. No. 3 (product of Toyo Jozo Co.) has a relative activity of approx. 10 ~ 15 % when the coenzyme thio-NAD is used, as compared to use of NAD. Optimum pH is approx. 9.5 for the forward reaction and approx. 7 ~ 7.5 for reverse reaction. The enzyme can utilize both NAD group and NADP group as coenzyme.

Myo-inositol in a specimen can be assayed by adding 0.001 ~ 0.5 ml of a specimen to the assay composition containing the above components (1) - (3), components (1) - (4) or components (1) - (3) and (5), reacting at approx. 37 °C, then measuring the amount of generated $A_2$ or consumed $B_1$ over an interval spanning two time points after starting the reaction. For example a minute between 3 mins. and 4 mins. after starting, or for five minutes between 3 mins. and 8 mins. after starting the reaction. Measurement is effected by determining the changes of absorption at each optical absorption. For example, when $A_2$ is thio-NADH and $B_1$ is NADH, the generated $A_2$ is measured by an increase of absorption at 400 nm or consumed $B_1$ is measured by a decrease of absorption at 340 nm and the thus-obtained value is compared with the value of a known concentration of reference myo-inositol. Thus the concentration of myo-inositol in a specimen can be measured in real time.

According to the assay method where myo-inositol itself existing in a specimen is introduced into the enzymatic cycling reaction, any coexisting substances in the specimens do not affect the result. Hence a measurement of a blank value of the specimen is not required. Thus, a simple assay system using a rate assay can be achieved.

The measurement of a value of $A_2$ or $B_1$ can be performed by absorbancy, or alternatively by other known analytical methods.

As explained above, no measurement error can occur, due to the use of coenzymes each having a different absorption in its reduced form. The amounts of myo-inositol can also be assayed precisely and rapidly even with a small amount of specimen, due to combining enzymatic cycling reaction.

## Examples

The following examples illustrate the present invention

### Example 1

Reagents:

40 mM Glycine-NaOH buffer (pH 10.0)
2 mM Thio-NAD (Sankyo Co.)

0.2 mM reduced NAD (Oriental Yeast Co.)
150 U/ml Myo-inositol dehydrogenase (Toyo Jozo Co. Bacillus sp. No. 3)

Procedure:

The above reagent mixture (1 ml) was put into cuvette and 20 µl of each of a range of concentrations of myo-inositol solution (0, 10, 20, 30, 40 and 50 µM, respectively) was added thereto, with the reaction temperature at 37°C. After incubation commenced, a difference in absorbance at 400 nm at 2 mins. and 7 mins. was measured. The results are shown in Fig. 5, from which it can be seen that a linear relation between the amount of myo-inositol and the change in absorption was observed.

Example 2

Reagents:

40 mM Glycine-NaOH buffer (pH 9.5)
2 mM Thio-NAD (Sankyo Co.)
0.1 mM reduced deamino NAD (Sigma Co.)
200 U/ml Myo-inositol dehydrogenase (Toyo Jozo Co. Bacillus sp. No. 3)

Procedure:

The above reagent mixture (1 ml) was put into a cuvette and 50 µl of each of a range of concentrations of myo-inositol solution (0, 2, 4, 6, 8 and 10 µM, respectively) was added thereto, then the mixture was incubated at 37 °C for 60 minutes. Then the reaction was stopped by adding 0.5 % sodium dodecyl sulfate (1 ml). Absorbance at 400 nm was measured. The results are shown in Fig. 6, from which it can be seen good linear quantitative curve.

Example 3

Reagents:

50 mM Glycine-NaOH buffer (pH 10.0)
0.2 mM reduced NAD (Oriental yeast Co.)
4 mM thio-NAD (Sankyo Co.)
250 U/ml Myo-inositol dehydrogenase (Toyo Jozo Co. Bacillus sp. No. 3)
0.2 % Triton X - 200

Procedure:

The above reagent mixture (1 ml) was put into cuvette and 20 µl of each of three different serum was added thereto, with the reaction temperature at 37 °C. After incubation commenced, a difference in absorbance at 400 nm at 5 min. and 6 min. was measured.

50 µM myo-inositol solution (standard solution) and distilled water (reagent blank) were treated in the same manner as above and an amount of myo-inositol in serum samples was calculated from the difference in absorbance between the standard solution and samples.

In table hereinbelow, the results obtained from three different serum are shown.

| | Difference Absorbance (mAbs) | Myo-inositol concentration |
|---|---|---|
| Reagent blank | 2 | - |
| Standard solution | 29 | 50 µM |
| Serum 1 | 25 | 42.6 µM |

(continued)

| | Difference Absorbance (mAbs) | Myo-inositol concentration |
|---|---|---|
| Serum 2 | 21 | 35.2 μM |
| Serum 3 | 31 | 53.7 μM |

Example 4

Reagents:

40 mM Glycine-NaOH buffer (pH 10.0)
15 mM NADP (Oriental yeast Co.)
50 μM thio-NAD (Sankyo Co.)
0.4 M Ethanol
30 U/ml Alcohol dehydrogenase (Oriental yeast Co.)
250 U/ml Myo-inositol dehydrogenase (Toyo Jozo Co. Bacillus sp. No. 3)

Procedure:

The above reagent mixture (1 ml) was put into cuvettes and 50 μl of each of concentrations of myo-inositol solution (0, 20, 40, 60, 80 and 100 μM, respectively) was added thereto, with the reaction temperature at 37 °C. After incubation commenced, a difference in absorbancy at 340 nm at 3 mins and 8 mins. was measured. The results are shown in Fig. 7.

Example 5

Reagents:

50 mM Phosphate buffer (pH 7.0)
0.25 mM reduced NADP (Oriental yeast Co.)
50 μM thio-NAD (Sankyo Co.)
5 mM Dihydroxyacetone phosphate
10 U/ml Glycerol-3-phosphate dehydrogenase (Boehringer Mannheim, rabbit muscle)
250 U/ml Myo-inositol dehydrogenase (Toyo Jozo Co. Bacillus sp. No. 3)

Procedure:

The above reagent mixture (1 ml) was put into cuvettes and 50 μl of each of concentrations of myo-inositol solution (0, 50, 100, 150, 200 and 250 μM, respectively) was added thereto, with the reaction temperature at 37 °C . After incubation commenced, a difference in absorbance at 340 nm at 3 mins. and 8 mins. was measured. The results are shown in Fig. 8.

Example 6

| Culture Bacillus sp. No. 3: | |
|---|---|
| Yeast extract (Kyokuto Seiyaku Co.) | 2 % |
| Peptone (Kyokuto Seiyaku Co.) | 2 % |
| $K_2HPO_4$ (Wako Pure Chem. Co.) | 0.2 % |
| $CaCl_2$ (Wako Pure Chem. Co.) | 0.02 % |

(continued)

| Culture Bacillus sp. No. 3: | |
|---|---|
| $MgSO_4 \cdot 7H_2O$ (Wako Pure Chem. Co.) | 0.05 % |
| Myo-inositol (Wako Pure Chem. Co.) | 2 % |
| pH 7.3 | |

100 ml of a liquid medium comprising the above composition was sterilized in a 500 ml Erlenmeyer flask at 120 °C for 20 mins. One loopful of Bacillus sp. No. 3 was inoculated into the medium and the medium was cultured at 50 °C with stirring at 120 r.p.m. for 30 hours to obtain the cultured mass (85 ml) (enzyme activity: 1.2 U/ml). 20 1 of a liquid medium comprising the above composition added with disform CB 442 (Nihon Yushi Co.) 0.1 % was sterilized in a 30 l jar fermenter by heating. 85 ml of the pre-cultured seed culture obtained in the step above was inoculated therein and the mixture was cultured at 50°C, with aeration of 20 l/ml., inner pressure 0.4 kg/$cm^2$, and agitation at 150 r.p.m. for 24 hours to obtain the cultured mass (18.0 1) (enzyme activity: 1.8 U/ml).

Example 7

Purification of enzyme

Bacterial cells collected by centrifugation from the cultured broth obtained in Example 6 were suspended in 20 mM phosphate buffer (pH 7.5, 5 1) containing 0.1% lysozyme (Eizai Co.) and solubilized at 37C for 1 hour; then the mixture was centrifuged to remove precipitate and to obtain a supernatant solution (4500 ml) (activity: 6 U/ml). Acetone (1.8 1) was added to the supernatant solution to separarte the precipitate, which was dissolved in 20 mM phosphate buffer to obtain crude extract (1 lit., 24.2 U/ml).

Ammonium sulfate (200 g) was added to the solution, Which was mixed well by stirring and then centrifuged to separate the precipitate. An additional 250 g ammonium sulfate was then added to the supernatant solution, and the solution was centrifuged to obtain a new precipitate. The new precipitate was dissolved in 20 mM phosphate buffer (pH 7.5) to obtain enzyme solution (500 ml, specific activity 36.3 U/ml), and the resultant solution was dialyzed overnight against 20 mM phosphate buffer (pH 7.5, 20 lit.). The dialyzed enzyme solution was charged on a column of DEAE-Sepharose CL-6B (Pharmacia Co.)(250 ml) which was buffered with 20 mM phosphate buffer (pH 7.5), washed with 20 mM phosphate buffer containing 0.1 M KCl, (pH 7.5, 1 lit) and eluted with 20 mM phosphate buffer containing 0.3 M KCl (pH 7.5) to obtain an enzyme solution (350 ml, activity 35.2 U/ml). The enzyme solution was dialyzed overnight against 10 mM phosphate buffer (pH 7.0, 20 lit.) Bovine serum albumin (Sigma Co., 0.2 g) was dissolved in the thus-obtained enzyme solution, then the solution was lyophilized to obtain the lyophilized enzyme (1.1 g, 10.6 U/mg).

## Claims

1. A myo-inositol dehydrogenase which has the following properties:

(1) substrate specificity for myo-inositol and catalyses the reaction:

$$\text{myo-inositol} + \text{NAD} \leftrightharpoons \text{myo-inosose} + \text{reduced NADH}$$

(2) molecular weight: 130,000 ± 15,000 (gelfiltration method by TSK gel G 3000 SW)
(3) isoelectric point: pH 4.5 ± 0.5
(4) Km-value:

| | |
|---|---|
| Km value for myo-inositol | 0.64 mM |
| Km value for NAD | 0.004 mM |

(5) optimum pH: approx. pH 9.5
(6) pH-stability: more than 80% of remaining activity at a pH of from 6.5 to 9.0
(7) heat stability: more than 95% of remaining activity at temperatures up to 60°C.

2. A process for the production of a myo-inositol dehydrogenase as defined in claim 1 which comprises culturing a

myo-inositol dehydrogenase producing microorganism which is Bacillus sp. No. 3 (FERM BP-3013) or a mutant thereof, and isolating said myo-inositol dehydrogenase from the cultured mass.

3. Bacillus sp. No. 3 (FERM BP-3013) or a mutant thereof which is capable of producing a myo-inositol dehydrogenase as defined in claim 1.

4. A culture of Bacillus sp. No. 3 (FERM BP-3013), or a mutant thereof which is capable of producing a myo-inositol dehydrogenase as defined in claim 1, in a culture medium which comprises a source of assimilable carbon, a source of assimilable nitrogen and mineral salts and which is free of other microorganisms.

## Patentansprüche

1. Myoinositoldehydrogenase, die die folgenden Eigenschaften aufweist:

(1) Sie weist eine Substrat Spezifität für Myoinositol auf und katalysiert die Reaktion:

Myoinositol + NAD ⇔ Myoinosose + reduziertes NADH

(2) Molekulargewicht: 130.000 ± 15.000 (Gelfiltrationsverfahren mit TSK Gel G 3000 SW)
(3) Isoelektrischer Punkt: pH 4,5 ± 0,5
(4) Km-Wert:

| | |
|---|---|
| Km-Wert für Myoinositol | 0,64 mM |
| Km-Wert für NAD | 0,004 mM |

(5) Optimaler pH: etwa pH 9,5
(6) pH-Stabilitat: mehr als 80 % Restaktivität bei einem pH von 6,5 bis 9,0.
(7) Hitzestabilität: mehr als 95 % Restaktivität bei Temperaturen von bis zu 60° Celsius.

2. Verfahren zur Herstellung einer Myoinositoldehydrogenase nach Anspruch 1, das das Züchten eines Myoinositoldehydrogenase produzierenden Mikroorganismus, welcher der Bacillus sp. Nr. 3 (FERM BP-3013) oder eine Mutante davon ist, und Isolieren der Myoinositoldehydrogenase aus der Kulturmasse umfaßt.

3. Bacillus sp. Nr. 3 (FERM BP-3013) oder eine Mutante davon, die eine Myoinositoldehydrogenase nach Anspruch 1 produzieren kann.

4. Kultur von Bacillus sp. Nr. 3 (FERM BP-3013) oder einer Mutante davon, die eine Myoinositoldehyrogenase nach Anspruch 1 produzieren kann, in einem Kulturmedium, das eine Quelle von assimilierbarem Kohlenstoff, eine Quelle von assimilierbarem Stickstoff und Mineralsalze enthält und das frei von anderen Mirkoorganismen ist.

## Revendications

1. Myo-inositol déhydrogènase qui a les propriétés suivantes :

(1) une spécificité au substrat pour le myo-inositol et catalysant la réaction :

myo-inositol + NAD ⇆ myo-inosose + NADH réduit

(2) masse moléculaire : 130.000 ± 15.000 (méthode par filtration de gel par un gel TSK G 3000 SW)
(3) point isoélectrique : pH 4,5 ± 0,5
(4) Indice Km :

| | |
|---|---|
| Indice Km pour le myo-inositol | 0,64 mM |

(suite)

| | |
|---|---|
| Indice Km pour le NAD | 0,004 mM |

(5) pH optimum : approximativement pH 9,5
(6) stabilité au pH : plus de 80 % de l'activité subsiste à un pH de 6,5 à 9,0
(7) stabilité thermique : plus de 95 % de l'activité subsiste à une température allant jusqu'à 60°C.

**2.** Procédé de préparation d'une myo-inositol déhydrogènase telle que définie à la revendication 1, qui consiste à cultiver un micro-organisme produisant de la myo-inositol déhydrogènase, qui est Bacillus sp. No. 3 (FERM BP-3013) ou l'un de ses mutants, et à isoler la myo-inositol déhydrogènase de la masse de culture.

**3.** Bacillus sp. No.3 (FERM BP-3013) ou l'un de ses mutants qui est capable de produire de la myo-inositol déhydrogènase, telle que définie à la revendication 1.

**4.** Culture de Bacillus sp. No. 3 (FERM BP-3013) ou l'un de ses mutants qui est capable de produire une myo-inositol déhydrogènase, telle que définie à la revendication 1, dans un milieu de culture qui comprend une source de carbone assimilable, une source d'azote assimilable et des sels minéraux et qui est exempte d'autres micro-organismes.

FIG. 1

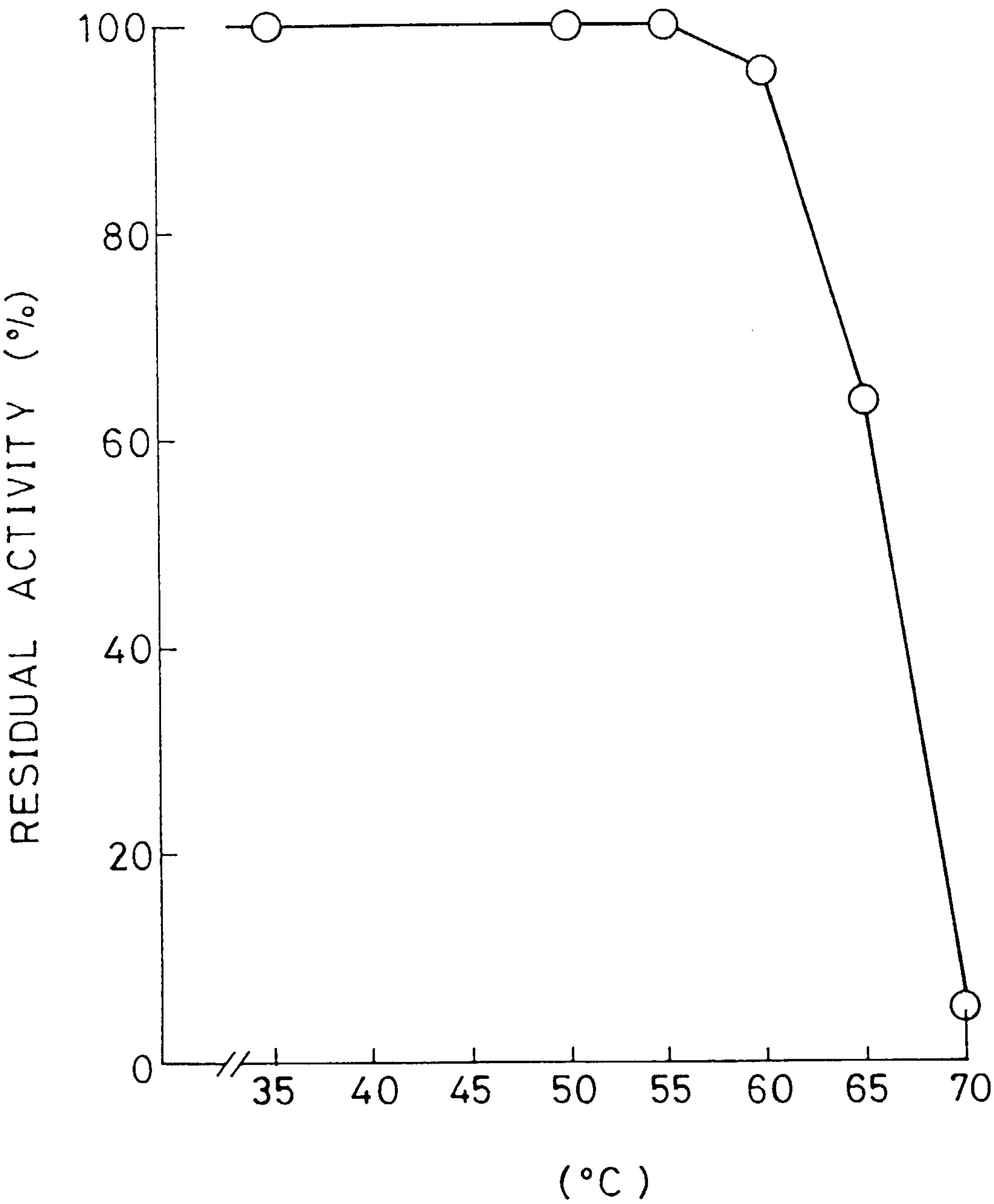

FIG. 2

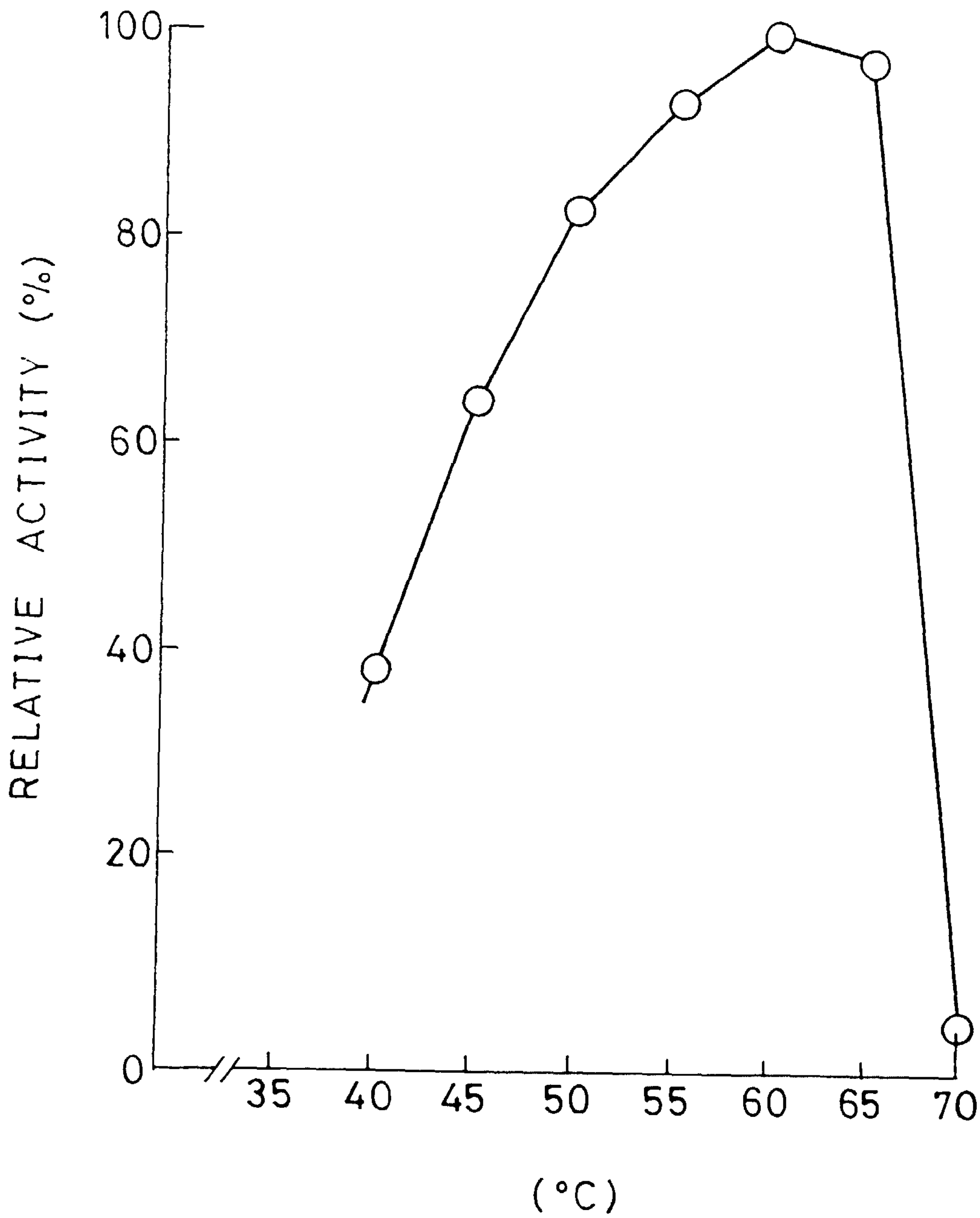

FIG. 3

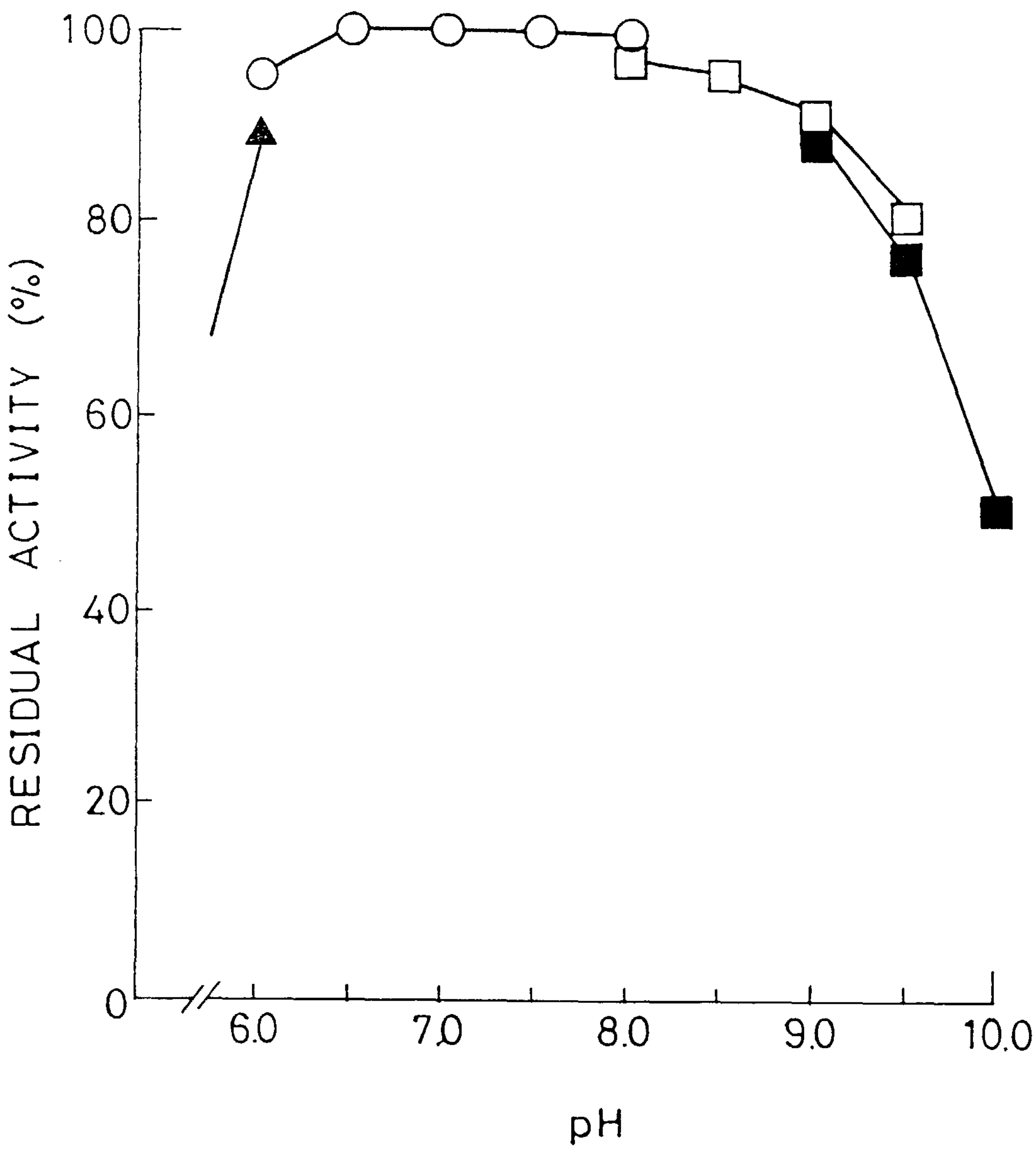

FIG. 4

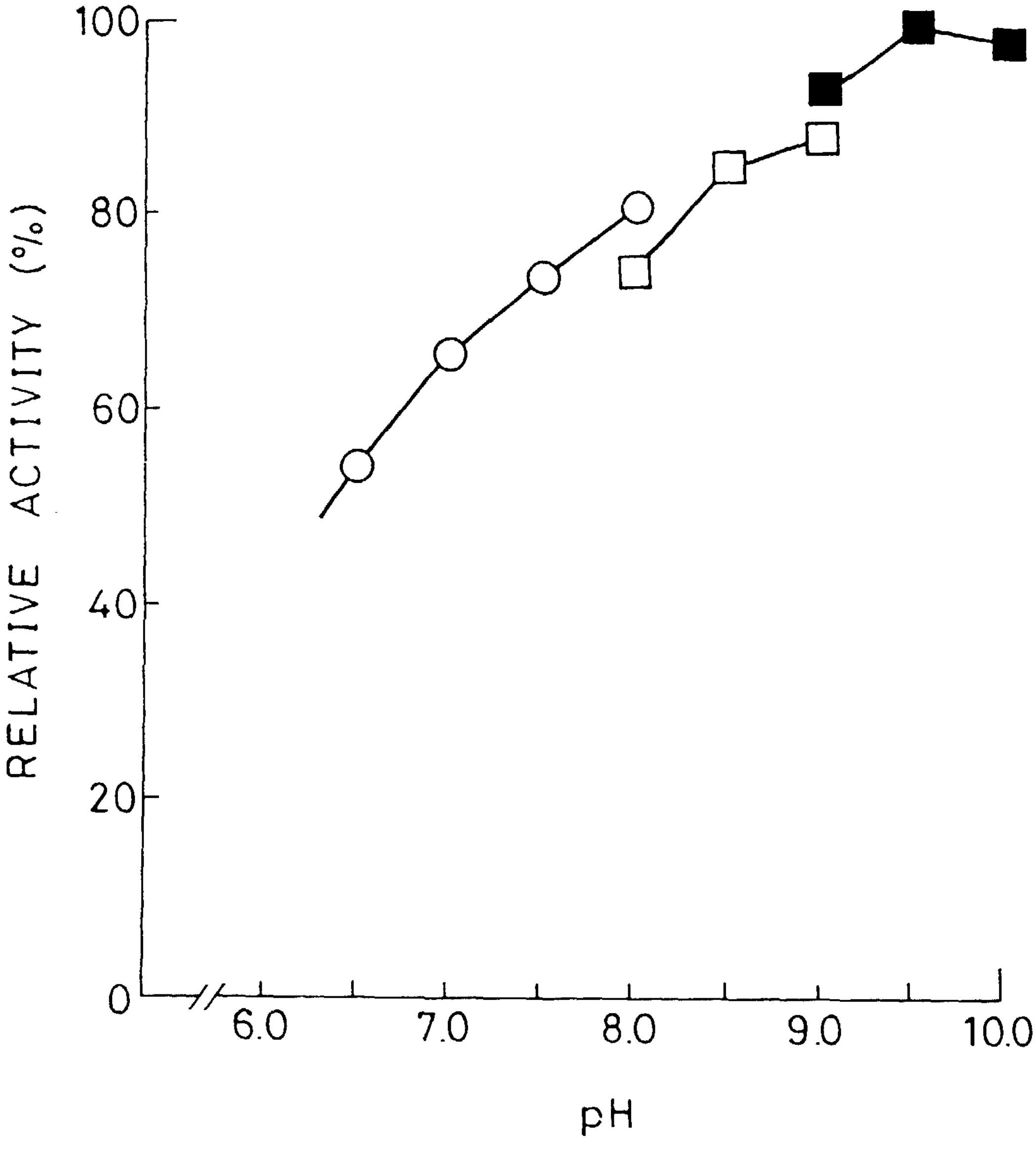

FIG. 5

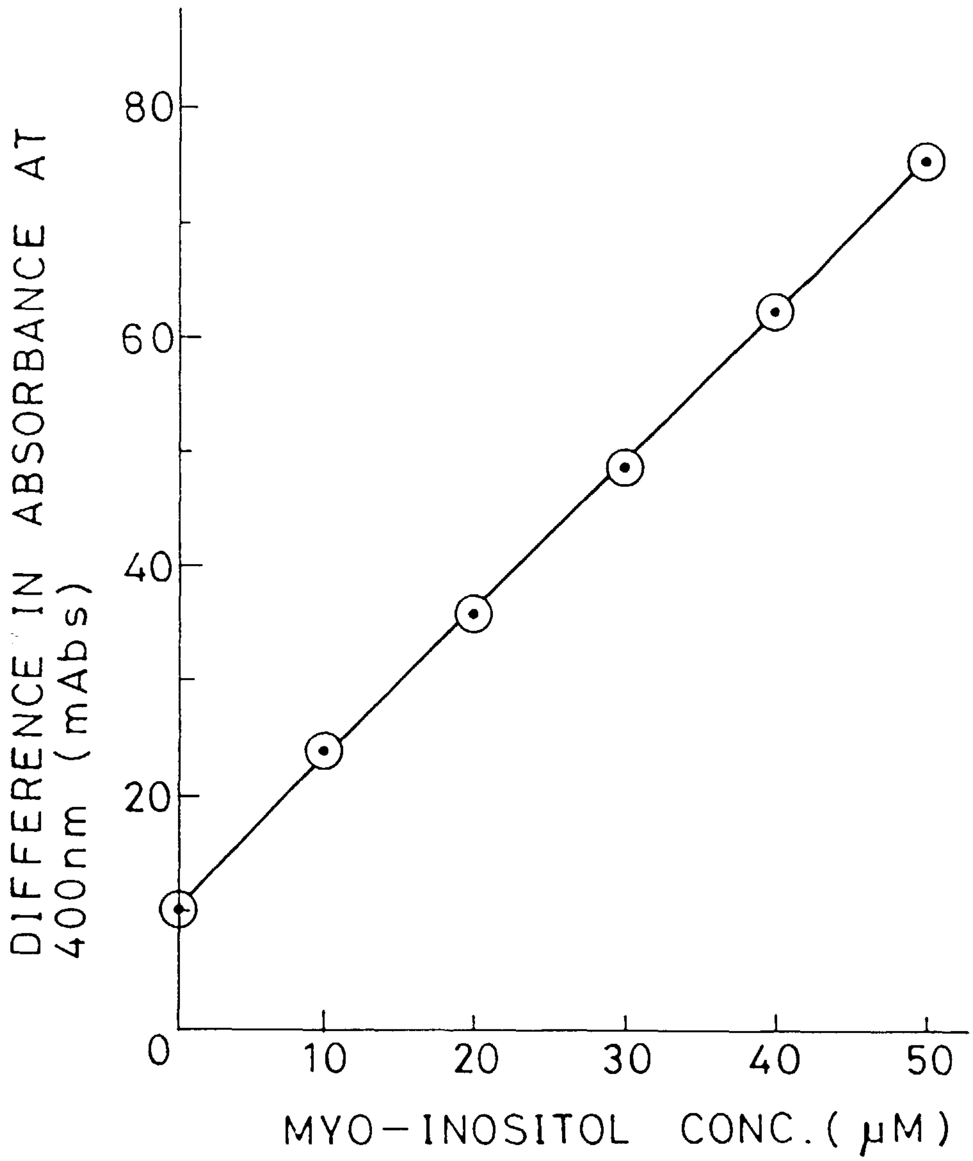
DIFFERENCE IN ABSORBANCE AT 400nm (mAbs)
80
60
40
20
0
10
20
30
40
50
MYO-INOSITOL CONC. ( μM )

FIG. 6

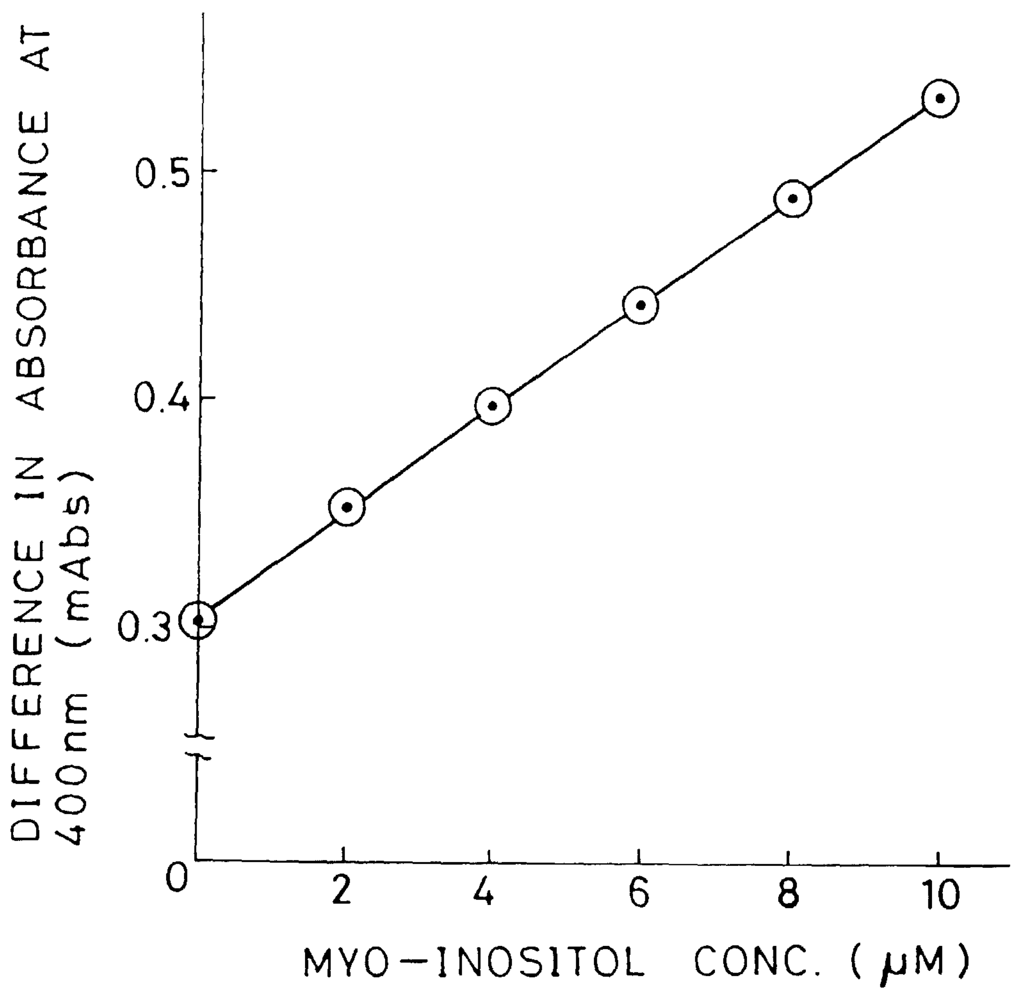
DIFFERENCE IN ABSORBANCE AT 400nm (mAbs)
0.5
0.4
0.3
0
2
4
6
8
10
MYO-INOSITOL CONC. (μM)

FIG. 7

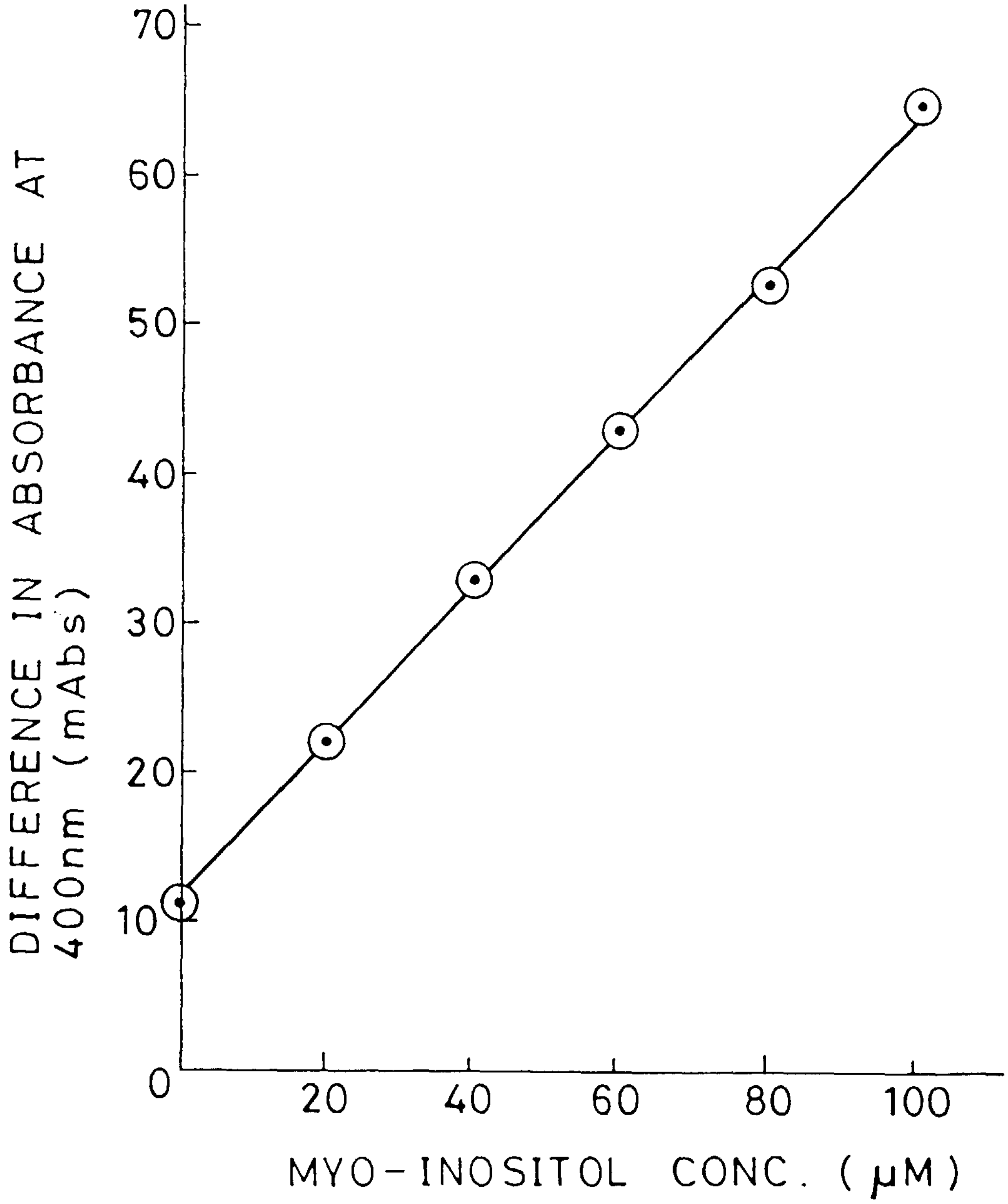
70
60
50
40
30
20
10
0
20
40
60
80
100
DIFFERENCE IN ABSORBANCE AT 400nm (mAbs)
MYO-INOSITOL CONC. (μM)

FIG. 8

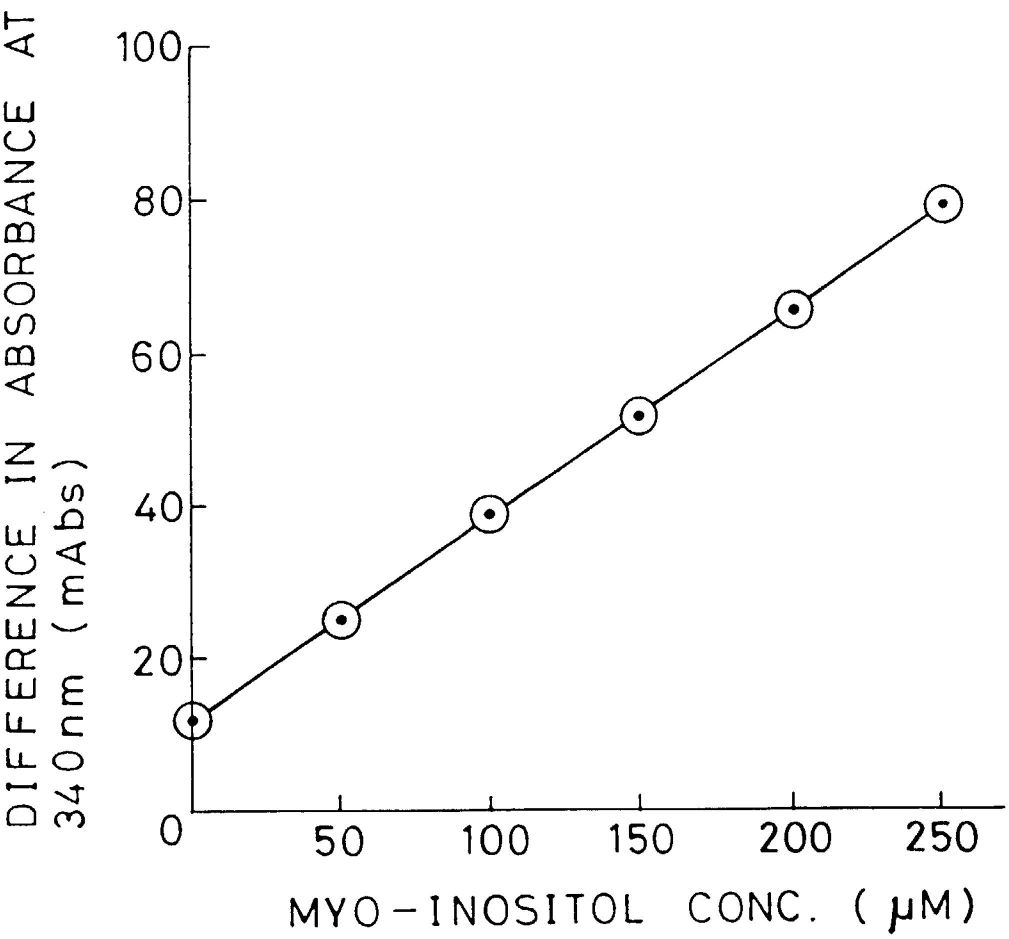
DIFFERENCE IN ABSORBANCE AT 340nm (mAbs)
100
80
60
40
20
0
50
100
150
200
250
MYO-INOSITOL CONC. (μM)